(19) **Europäisches Patentamt**

**European Patent Office**

**Office européen des brevets**

(11) **EP 1 123 417 B1**

(12) **EUROPÄISCHE PATENTSCHRIFT**

(45) Veröffentlichungstag und Bekanntmachung des Hinweises auf die Patenterteilung:
**23.04.2003 Patentblatt 2003/17**

(51) Int Cl.7: **C12Q 1/68**

(21) Anmeldenummer: **99953906.7**

(86) Internationale Anmeldenummer:
**PCT/EP99/07986**

(22) Anmeldetag: **21.10.1999**

(87) Internationale Veröffentlichungsnummer:
**WO 00/023616 (27.04.2000 Gazette 2000/17)**

(54) **VERFAHREN ZUR EXPONENTIELLEN AMPLIFIKATION MOLEKULARER MATRIZEN**

METHOD FOR THE EXPONENTIAL AMPLIFICATION OF MOLECULAR MATRICES

PROCEDE POUR L'AMPLIFICATION EXPONENTIELLE DE MATRICES MOLECULAIRES

(84) Benannte Vertragsstaaten:
**AT BE CH CY DE DK ES FI FR GB GR IE IT LI LU MC NL PT SE**

(30) Priorität: **21.10.1998 DE 19848403**
**19.11.1998 DE 19853185**

(43) Veröffentlichungstag der Anmeldung:
**16.08.2001 Patentblatt 2001/33**

(73) Patentinhaber:
• **Noxxon Pharma AG**
**13355 Berlin (DE)**
• **Von Kiedrowski, Günter**
**44797 Bochum-Stiepel (DE)**

(72) Erfinder:
• **VON KIEDROWSKI, Günter**
**D-44797 Bochum-Stiepel (DE)**
• **KLUSSMANN, Sven**
**D-10709 Berlin (DE)**
• **KLEIN, Thomas**
**D-14109 Berlin (DE)**
• **FÜRSTE, Jens, Peter**
**D-13559 Berlin (DE)**

(74) Vertreter: **Helbing, Jörg, Dr. Dipl.-Chem. et al**
**Patentanwälte**
**von Kreisler-Selting-Werner,**
**Postfach 10 22 41**
**50462 Köln (DE)**

(56) Entgegenhaltungen:
EP-A- 0 374 665     WO-A-94/02515
WO-A-96/04404       WO-A-98/14610
WO-A-98/33941       WO-A-98/44151
US-A- 5 616 478     US-A- 5 629 158
US-A- 5 795 714

• FERRIS ET AL.: "SYNTHESIS OF LONG PREBIOTIC OLIGOMERS ON MINERAL SURFACES" NATURE, Bd. 381, 1996, Seiten 59-61, XP002134111
• LUTHER ET AL.: "SURFACE-PROMOTED REPLICATION AND EXPONENTIAL AMPLIFICATION OF DNA ANALOGUES" NATURE, Bd. 396, 19. November 1998 (1998-11-19), Seiten 245-248, XP002134112

SEQUENZPROTOKOLL

<110> von Kiedrowski, Günther NOXXON Pharma AG

<120> Verfahren zur exponentiellen Amplifikation molekularer Matrizen

<130> 992074wo/JH/ml

<140>

<141>

<160> 1

<170> PatentIn Ver. 2.1

<210> 1

<211> 14

<212> DNA

<213> Künstliche Sequenz

<220>

<223> Beschreibung der künstlichen Sequenz: reference matrix

<400> 1

`gaatccatgg taag`                                                            14

**Beschreibung**

Die vorliegende Erfindung betrifft ein Verfahren zur exponentiellen Amplifikation molekularer Matrizen, d. h. sequenzisomeren Verbindungen mit Templat-Eigenschaften. In dem Verfahren wird eine Produkthemmung durch Reaktionsführung an einer Festphasenoberfläche ausgeschaltet. Die vorliegende Erfindung betrifft weiterhin ein Verfahren zur chemischen Evolution unter Verwendung des genannten Verfahrens zur exponentiellen Amplifkation molekularer Matrizen.

Chemisch selbst-replizierende Systeme wurden entworfen, um die minimalen Anforderungen für eine molekulare Replikation zu identifizieren [D. Sievers et al., Self-Reprod. of Supramolecular Structures, Kluwer Publishers, Dordrecht (1994)], um das Prinzip auf synthetische supramolekulare Systeme zu übertragen [E.A. Wintner et al., Acc. Chem. Res. 27, 198-203 (1994)] und um zu einem besseren Verständnis der Reichweite und der Beschränkungen von Selbstorganisationsvorgängen [M. Eigen, Naturwissenschaften 58, 465-523 (1971)] zu gelangen, von denen man glaubt, daß sie für die Entstehung des Lebens auf der Erde relevant sind [L. Orgel, Acc. Chem. Res. 28, 109-118 (1995)]. Derzeitige Ausführungen verwenden Oligonucleotid-Analoga [G. von Kiedrowski, Angew. Chem. Int. Ed. Engl. 25, 932-935 (1986); W.S. Zielinski et al., Nature 327, 346-347 (1987); G. von Kiedrowski et al., Angew. Chem. Int. Ed. Engl. 30, 423-426, ibid. 892 (1991); T. Achilles et al., Angew. Chem. Int. Ed. Engl. 32, 1198-1201 (1993); D. Sievers et al., Nature 369, 221-224 (1994); T. Li et al., Nature 369, 218-221 (1994); B. Martin et al. Helv. Chim Acta 80, 1901-1951 (1997); D. Sievers et al., Chem. Eur. J. 4, 629-641 (1998)], Peptide [D. Lee et al., Nature 382, 525-528 (1996); K. Severin et al., Angew. Chem. Int. Ed. 37, 126-128 (1998); D.H. Lee et al., Nature 390, 591-594 (1997); S. Yao et al., Angew. Chem. Int. Ed. 37, 478-481 (1998); K.S. Severin et al. Chem. Eur. J. 3, 1017-1024 (1997)] und andere Moleküle [T. Tjivikua et al., J. Am. Chem. Soc. 112, 1249-1250 (1990); A. Terfort et al., Angew. Chem. Int. Ed. Engl. 31, 654-656 (1992); J.-I. Hong et al., Science 225, 848-850 (1992); Q. Feng et al., Science 256, 1179-1180 (1992); R.J. Pieters et al., Angew. Chem. Int. Ed. Engl. 106, 1579-1581 (1994); D.N. Reinhoudt et al., J. Am. Chem. Soc. 118, 6880-6889 (1996); B. Wang et al., Chem. Commun. 16, 1495-1496 (1997)] als Matrizen und beruhen entweder auf autokatalytischen, kreuzkatalytischen oder kollektiv katalytischen Wegen der Matrizenbildung. Ein häufiges Problem dieser Systeme ist die Produkthemmung, die zu einer parabolischen anstelle einer exponentiellen Amplifikation führt [G. von Kiedrowski, Bioorg. Chem. Front. 3, 113-146 (1993)]. Letztere ist die dynamische Voraussetzung für eine Selektion im Darwinschen Sinn [E. Szathmáry et al., J. Theor. Biol. 138, 55-58 (1989); R.W. Wills et al., Santa Fe Institute Working Paper 97-07-065 (1997)].

Mehrere Theorien der Entstehung des Lebens haben die Bedeutung von Oberflächen für die chemische Evolution hervorgehoben [J.D. Bernal, The Physical Base of Life, Routledge & Kegan Paul, London, 1951; A.G. Cairns-Smitz, The Life Puzzte, Oliever & Boyd, Edinburgh, 1971; H. Kuhn et al., Angew. Chem., Int. Ed. Engl. 20, 500-520 (1981); G. Wächtershäuser, Microbiol. Rev. 52, 452-484 (1988); E. Szathmáry et al., J. Theor. Biol. 187, 555-571 (1997); L. Orgel, Origins Life Evol. Biosphere 28, 227-234 (1998)]. So wurden Verfahren mit schrittweiser Zufuhr schon früher in zwei verschiedenen chemischen Systemen eingesetzt, die als Modelle für potentielle prabiotische Prozesse beschrieben wurden [T. Li et. al., Nature 369, 218-221 (1994); J. P. Ferris et al., Nature 381, 59-61 (1996); G. von Kiedrowski, Nature 381, 20-21 (1996)]. In Li et al. werden eine chemische Replikation von Duplex-DNA, die aus palindromischen (symmetrischen) Homopyrimidin- und Homopurin-Strängen bestand, erreicht. Der Homopyrimidin-Strang wurde über Dreifachhelix-Verknüpfung aus seinen Vorstufenfragmenten synthetisiert und diente dann als Matrize für die chemische Verknüpfung der Vorstufen des Homopurin-Stranges. Die schrittweise Zufuhr von Homopyrimidinund Homopurin-Fragmenten ermöglichte dabei, eine Komplexierung der Fragmente zu verhindern und daher zwischen den jeweiligen Triplex- und Duplex-Verknüpfungs-Zwischenstufen hin- und herzuschalten. Ferris et al. haben die Synthese von langen Oligonucleotid- und peptidartigen Materialien auf der Oberfläche von mineralischen Trägem nachgewiesen. In diesen Systemen erlaubte die schrittweise Zufuhr die Ergänzung aktivierter Vorstufen und ermöglichte es damit, den längenbeschränkenden Effekt der Vorstufenhydrolyse zu überwinden.

Ein Selektions- und Amplifikationsverfahren mit evolutivem Charakter ist weiterhin aus der WO 91/19813 bekannt. In diesem Verfahren wird eine Subpopulation einer Ausgangsmutantenbibliothek durch reversibles Binden (Partitionieren) an einer Zielstruktur erzeugt und die selektierten Mutanten nachfolgend amplifiziert. Bei einer solchen Amplifikation tritt jedoch häufig das auch z. B. bei der PCR bekannte Problem der Produkthemmung auf.

US 5,795,714 beschreibt ein Verfahren zur Replikation, jedoch nicht zur Amplifikation von Nukleinsäurenarrays. In dem hier beschriebenen Verfahren dienen immobilisierte Template zur Synthese komplementärer Stränge durch enzymatische Ligation von Fragmenten, von denen eines eine immobilisierbare Funktion besitzt (hier Biotin). Es erfolgt ein Transfer des synthetisierten Stranges auf eine zweite Oberfläche, die mit der immobilisierbaren Funktion reagiert (hier Avidin), und zwar im Sinne eines Thermoblotting-Vorgangs. Hierbei wird das ursprüngliche Masterarray - in dem genannten US-Patent handelt es sich um ein sogenanntes Pinarray, bei dem die Nukleinsäuren auf kleinen Plastikstiften immobilisiert sind - auf eine Membran kopiert, die ihrerseits nicht mehr kopiert werden kann.

Die Aufgabe der vorliegenden Erfindung bestand nun darin ein Amplifikationsverfahren für sequenzisomere Verbindungen mit Templateigenschaften (nachfolgend kurz "molekulare Matrizen") zur Verfügung zu stellen, das eine unbegrenzte Vervielfältigung der molekularen Matrize, d. h. ohne Produkthemmung ermöglicht.

Die Aufgabe der vorliegenden Erfindung bestand weiterhin darin, ein Verfahren zur Evolution molekularer Matrizen zur Verfügung zu stellen, in dem der Amplifikationsschritt keinen limitierenden Faktor darstellt, d. h., daß eine unbegrenzte Vervielfältigung der molekularen Matrize ohne Produkthemmung möglich ist.

Überraschenderweise wurde ein iteratives schrittweises Verfahren gefunden, das es erlaubt, die vorhandene Menge an molekularer Matrize exponentiell zu erhöhen. Das Verfahren verwendet dazu die Oberfläche eines festen Trägers. Durch chemische Verknüpfung auf immobilisierten Matrizen werden Kopien aus Vorstufenfragmenten synthetisiert, die dann freigesetzt und immobilisiert werden, so daß sie zu neuen Matrizen werden. Der Vorgang kann beliebig oft wiederholt werden.

Die vorliegende Erfindung betrifft'somit

    (I) ein Verfahren zur exponentiellen Amplifikation molekularer Matrizen, umfassend

        (a) Binden von molekularen Matrizen an die Oberfläche einer Festphase mittels eines reversiblen Linkers an der Matrize;
        (b) Zugabe von Matrizenfragmenten, wobei eines der Fragmente eine gegebenenfalls geschützte Linkereinheit aufweist;
        (c) Synthese von Kopien der Matrize;
        (d) Entfernen von überschüssigen Matrizenfragmenten und Reaktionshilfstoffen;
        (e) Ablösen der Kopien von den Matrizen;
        (f) Besiedlung freier Bindungsstellen an der Festphase durch synthetisierte Matrizenkopien; und
        (g) Beliebig häufige Wiederholung der Schritte (a) - (f) und

    (II) ein Verfahren zur chemischen Evolution durch exponentielle Amplifikation molekularer Matrizen, umfassend einen oder mehrere der Evolutionszyklen

        (1) Selektion einer Subpopulation molekularer Matrizen aus einer kombinatorischen Ausgangsbibliothek; und
        (2) Amplifikation und Mutation der selektiven Matrizen zur Bereitstellung einer Mutantenbibliothek, umfassend mehr als eine der in (I) definierten Amplifikationszyklen (a) bis (f).

Die Rolle der Festphase (nachfolgend auch "Träger") in den erfindungsgemäßen Verfahren besteht darin, komplementäre Matrizen, die in Lösung stabile Duplexe bilden würden, voneinander getrennt zu halten. Geeignete Trägermaterialien bestehen dabei aus organischem oder anorganischem Material oder aus einem Hybrid dieser Materialien. Organische Trägermaterialien sind Polymere auf Zuckerbasis, vorzugsweise Agarose, Cellulose sowie geeignete Derivate oder technische Polymere wie Polystyrol, Polyacrylat, Polyacrylnitril, Polyalkene oder Pfropfcopolymer (z. B. PS-PEG, PAN-PEG, PAN-PAG usw.). Anorganische Trägermaterialien können z. B. Glas, funktionalisiertes Hydroxyapatit sowie Metalle sein, wobei insbesondere der Goldoberfläche (infolge der Goldthiolatwechselwirkung) besondere Bedeutung zukommt. Als Hybride sind z. B. paramagnetische Beads denkbar.

In den erfindungsgemäßen Verfahren können dabei die in Schritt (e) erzeugten Matrizenkopien identisch oder komplementär zu den Ausgangsmatrizen sein. Unter "komplementär" im Sinne der Erfindung ist zu verstehen, daß die Kopie der Matrize sich von der Ausgangsmatrize unterscheidet, die Kopie dieser Kopie jedoch wieder identisch mit der Ausgangsmatrize ist. Nachfolgend wird dies auch kurz mit "(+)-Strang" und "(-)-Strang" bezeichnet. Im Falle der Identität von Ausgangsmatrizen und Matrizenkopien kann der nach der Besiedlung von freien Bindungsstellen (Schritt (f)) folgende nächste Reaktionszyklus im selben Reaktionsgefäß durchgeführt werden. Andererseits wird, falls die erzeugten Matrizenkopien komplementär zur Ausgangsmatrize sind, Schritt (f) vorzugsweise in einem zweiten Reaktionsgefäß erfolgen und erst Schritt (f) des nächsten Reaktionszyklus wieder mit den ursprünglichen Matrizenfragmenten zusammen erfolgen.

In einer bevorzugten Ausführungsform des Verfahrens (I) werden nach Binden von molekularen Matrizen an der Oberfläche der Festphase überschüssige Bindungsstellen an der Festphase blockiert. Dieses Blockieren ist dabei abhängig von der Art der Verküpfung der verwendeten Matrizenfragmente an die Festphase, d. h. von der Art des Linkers. Die Bindung des Linkers in dem erfindungsgemäßen Verfahren kann sowohl mittels kovalenter als auch nichtkovalenter Bindung erfolgen, wobei unter nichtkovalenter Bindung sowohl ionische als auch nichtionische Bindungssysteme und insbesondere Mitglieder von immunologischen Bindungspaaren wie Avidin/Streptavidin oder Antigen-Antikörper umfaßt sind. Grundsätzliche Voraussetzung für die Auswahl der geeigneten Linker ist jedoch, daß diese orthogonal zu den übrigen in dem System verwendeten chemischen Bindungen, insbesondere zu den chemischen Bindungen in den Matrizen selbst und zwischen Matrize und Matrizenkopie ist, so daß die Matrizen ohne Bindungsbruch von der Festphasenoberfläche wieder entfernt werden können, d. h., ein schaltbares Binden an die Festphase gewährleistet ist. Für die Blockierung der überschüssigen Bindungsstellen an der Festphase bedeutet dies, daß die Blok-

kierung entweder durch Umsetzung mit chemischen Reagenzien, die eine kovalente Bindung mit freien Bindungsstellen eingehen (z. B. bei freien Thiolgruppen an der Festphase: eine Umsetzung mit geeigneten Thiolreagenzien unter Ausbildung von Disulfiden) oder durch Umsetzung mit geeigneten nichtkovalenten Verbindungen die freien Bindungsstellen abgesättigt werden (z. B. bei freien Antigenen in der Festphase eine Umsetzung mit den entsprechenden Antikörpern), erfolgen kann. Der Linker kann sich dabei an jedem beliebigen Ende der Matrize befinden, d. h. er kann bei einem Oligonukleotid sowohl am 5'- als auch am 3'-Ende sein und bei einem Peptid sowohl am N- als auch am C-Terminus.

Weiterhin erfordert die Besiedlung freier Bindungsstellen an der Festphase durch synthetisierte Matrizenkopien, daß freie Bindungsstellen für den nächsten Reaktionszyklus vorhanden sind. Dies kann zum einen durch Freisetzen neuer Bindungsstellen an der bereits in der Synthese vorhandenen Festphase erfolgen, andererseits aber auch durch Zugabe von neuem Festphasenmaterial erfolgen, bzw. durch Transfer der gebildeten Matrizenkopie auf neues Festmaterial. Von diesen drei Möglichkeiten sind die ersten beiden besonders für die Situation geeignet, wo Ausgangsmatrize und Matrizenkopie identisch sind. Die dritte Möglichkeit ist eher für solche Systeme geeignet, in denen die erzeugte Matrize komplementär zu der Ausgangsmatrize ist

In einer anderen Ausführungsform des Verfahrens (I) können geschützte Linkereinheiten der Matrizenkopien an dieser Stelle des Reaktionszyklus (d. h. vor, während oder nach Schritt (e) entschützt werden. Nach dem Entschützen reagieren diese dann mit freien Bindungsstellen an dem Trägermaterial. Bei dieser Ausführungsform können auch während der Schritte (b) - (e) freie Bindungsstellen an der Festphase vorliegen. Ein Blockieren überschüssiger Bindungsstellen nach dem Schritt (a) ist bei dieser Ausführungsform nicht notwendig.

Für die Art dieser Linkerschutzgruppen gelten die gleichen Voraussetzungen wie vorstehend für die Linkerchemie. Es muß auch hier Orthogonalität zu den übrigen chemischen Verbindungen gewährleistet sein. Für das jeweilige Amplifikationssystem geeignete Schutzgruppen sind z. B. T.W. Green, Protective Groups in Organic Synthesis, Wiley & Sons, N.J. zu entnehmen.

Eine besondere Ausführungsform besteht schließlich darin, daß anstelle von einer Immobilisierungsmethode zwei oder mehrere orthogonale Methoden zum Einsatz kommen. Dies impliziert dann, daß anstelle von einer Oberfläche zwei oder mehrere Oberflächen verwendet werden. Vorzugsweise wird der (+)-Strang über eine andere Methode immobilisiert als der (-) Strang, womit zwei orthogonale Immobilisierungsverfahren an zwei entsprechen funktionalisierten Oberflächen durchzuführen sind. Orthogonale Immobilisierungsverfahren können auf verschiedenen spezifischen nicht-kovalenten Wechselwirkungen aufbauen, z.B. auf dem Biotin-Avidin-Paar und dem Digoxygenin-Antigigoxygenin-Paar, aber auch auf orthogonalen kovalenten Verknüpfungsverfahren, wie z.B. Diels-Alder-Reaktionen und Kondensationsreaktionen. Bezeichnet man mit A-A' und B-B' zwei orthogonale kovalente oder nicht-kovalente Wechselwirkungen, so besteht das Replikationsverfahren darin, daß

(1a) der A'-funktionalisierte (+)-Strang an Oberfläche A immobilisiert wird,
(1b) der B'-funktionalisierte (-)-Strang an Oberfläche B immobilisiert wird,
(2a) zum (+)-Strang Fragmente hinzugefügt werden, von denen eines mit B' funktionalisiert ist,
(2b) zum (-)-Strang Fragmente hinzugefügt werden, von denen eines mit A' funktionalisiert ist,
(3a) die Ligation der Fragmente am (+)-Strang erfolgt,
(3b) die Ligation der Fragmente am (-)-Strang erfolgt,
(4a) die B'-funktionalisierte Kopie des (+)-Strangs abgelöst wird,
(4b) die A'-funktionalisierte Kopie des (-)-Strangs abgelöst wird.

In der bevorzugten technischen Ausführungsform verbleibt die A-Oberfläche stets von der B-Oberfläche getrennt. Die Teilschritte (a) und (b) in den Reaktionen (1a,b), (3a,b) und (4a,b) können dann für beide Oberflächen jeweils gemeinsam, d.h. in der gleichen Flüssigphase durchgeführt werden. Lediglich die Teilschritte (2a) und (2b) müssen getrennt ablaufen, da hier verhindert werden muß, dass das A'-Fragment mit der A-Oberfläche und das B'-Fragment mit der B-Oberfläche in Kontakt tritt.

In den erfindungsgemäßen Verfahren können Matrizen mit einer Länge von 2 bis 2000, insbesondere von 4 bis 50 Monomereinheiten verwendet werden. Als "Matrizenfragmente" in Schritt (b) des erfindungsgemäßen Verfahrens können sowohl einzelne Monomereinheiten als auch Oligomere dieser Monomereinheiten eingesetzt werden. Voraussetzung ist jedoch, daß eines dieser Monomereinheiten oder Oligomereinheiten eine geschützte Linkereinheit aufweist, wobei die Art der Linkerschutzgruppe wie vorstehend definiert ist.

Die Konzentration eingesetzter Matrizen im erfindungsgemäßen Verfahren ist abhängig von dem verwendeten Festphasenmaterial, insbesondere der Größe der Partikel und der Anzahl der Bindungsstellen an dessen Oberfläche. Ein weiterer begrenzender Faktor ist die Länge der Matrizen. Im allgemeinen sollte die Konstruktion der Matrizenmoleküle in der Reaktionsmischung von $10^{-15}$ bis $10^{-1}$, insbesondere von $10^{-12}$ bis $10^{-3}$ mol/l betragen. Die verwendeten Matrizensegmente sollten in einer Konzentration von $10^{-12}$ bis 1 mol/l, insbesondere von $10^{-9}$ bis $10^{-1}$ mol/l eingesetzt werden, wobei ein gewisser Überschuß an Matrizenfragmenten, bezogen auf die für die entsprechende Matrize benötigten Monomereinheiten eingesetzt wird. Besonders bevorzugt ist ein Überschuß von 1 : 1,1 bis 1 : 100 der Monomereinheiten, wobei bei einem Überschuß von über 10 die Reaktion unökonomisch ist.

Unter molekulare Matrizen im Sinne der vorliegenden Erfindung sind alle sequenzenisomeren Verbindungen, die Templat-Eigenschaften aufweisen, zu verstehen. Dies sind insbesondere Oligonukleotide und Oligonukleotidabkömmlinge (inklusive PNA, pRNA, 2'-5' Nukleotide und RNA/DNA-Spiegelmere), Oligopeptide und Oligopeptidabkömmlinge (insbesondere in coiled-coil (z. B. Leucin-Zipper) und Faltblatt-Arrangements). Bei den Oligonukleotid- und Oligopeptidderivaten sind insbesondere nicht natürliche Strukturen zu nennen, wie Oligonukleotide mit 2' - 5' Strukturen und Peptide mit D-Aminosäuren, die eine höhere Stabilität gegen spaltende Enzyme aufweisen. Als Templat-Rückgrat können vorzugsweise Phosphorsäurediester, -amidate, -thioate, -pyrrophosphate und andere Phosphorsäurederivate sowie Amide, aber auch Ester, Harnstoffe, Urethane, Disulfide, Imine, Acetale und C-C-Verknüpfungen verwendet werden. Die in den Templaten stattfindende molekulare Erkennung kann über Heterozyklen (insbesondere Nukleobasen), Salzbrücken (insbesondere Amidiniumcarboxylat-Wechselwirkung), Ion-Ion-Wechselwirkung, Ion-Dipol-Wechselwirkung, Dipol-Dipol-Wechselwirkung, Einschlußkomplexierung (inbesondere via Cyclodextrine und sogenannte molekulare Pinzetten), Stapel- und Chargetransfer-Wechselwirkung (insbesondere via Stapelung elektronenreicher und elektronenarmer Aromate) sowie über Ligand-Metall-Ligand-Wechselwirkung erfolgen. Die Template können sowohl lineare, zyklische, verzweigte (dendrimerartige) sowie zweidimensionale Topologie besitzen.

Geeignete Lösungsmittel für das erfindungsgemäße Verfahren sind Wasser und organische Reaktionsmedien oder Gemische derselben. Falls die Matrizen und Matrizenfragmente ionische Gruppen enthalten (wie Phosphorsäuremono- oder -dieester), sind wäßrige Lösungsmittelsysteme besonders bevorzugt. Die Lösungsmittel können weiterhin geeignete Puffersysteme enthalten.

Die Bindungsverknüpfung in Schritt (c) kann sowohl chemisch oder enzymatisch efolgen. Bei der chemischen Verknüpfung erfolgt dabei die Umsetzung der Reaktionspartner mit für die jeweilige Bindungsknüpfung geeigneten Aktivierungsreagenzien und Kondensationsmitteln. So erfolgt die Kondensationsreaktion von Phosphorsäurederivaten bei Reaktionsführung in wäßrigen Medien vorzugsweise unter Verwendung von wasserlöslichen Carbodiimiden. Für die enzymatische Verknüpfung können alle Enzyme verwendet werden, die die entsprechenden katalytischen Eingenschaften aufweisen (Ligasen, Polymerasen, Esterasen usw.).

Die Reaktionstemperatur für die vorstehend genannten Reaktionsschritte, insbesondere dem Verknüpfungsschritt (c), kann dabei je nach Reaktionsystem zwischen -20 und 100 °C liegen. Besonders bevorzugt sind Temperaturen zwischen 0 und 50 °C. Insbesondere für die Verknüpfungsreaktion mittels Polymerasen ermöglicht das erfindungsgemäße Amplifikationssystem eine Reaktionsführung unterhalb von 50 °C.

Nach der Synthese der Matrizenkopien wird in Schritt (d) überschüssige Matrizenfragmente und Reaktionshilfsstoffe (d. h. Kondensationsmittel, Aktivierungsreagentien, Puffer, Enzyme usw.) entfernt, die für die Verknüpfung benötigt wurden.

Das Ablösen der Matrizenkopien von den Matrizen ist abhängig von dem jeweiligen Matrizensystem und muß so erfolgen, daß sowohl die geknüpften chemischen Verbindungen als auch die Verknüpfung des Linkers mit Oberflächen bzw. geeignete Linkerschutzgruppen an den Matrizenkopien unbeeinträchtigt bleiben. Geeignete Maßnahmen sind z. B. Behandlungen von wäßrigen oder organisch wäßrigen Lösungen mit Denaturierungsreagenzien, Temperaturerhöhung, elektrische und magnetische Felder, mechanische Verfahren und Kombinationen derselben. Die vorliegende Erfindung bietet somit eine Vielzahl von Denaturierungsmöglichkeiten, d. h. ein Erhitzen auf über 90 °C wie bei TAQ-Polymerase in der PCR ist nicht zwingend erforderlich.

In dem Verfahren (II) erfolgt die Selektion der Subpopulation molekularer Matrizen durch eine reversible Bindung an eine oder mehrere Zielstrukturen (Targetmoleküte). Dies erfolgt vorzugsweise durch eine Adsorptionschromatographie. In dem erfindungsgemäßen Evolutionszyklus wird, falls die Ausgangsmutantenbibliothek keinen geeigneten Linker aufweist, in einem zusätzlichen Schritt (1') die selektierten molekularen Matrizen mit einem reversiblen Linker versehen. Die in Schritt (2) des Verfahrens erhaltene Mutantenbibliothek ist die Ausgangsbibliothek für den nächsten Evolutionszyklus. Die Mutation im Amplifikationszyklus wird durch die Reaktionsparameter in den Schritten (b) und (c) gesteuert. Die Anzahl der Evolutionszyklen hängt dabei zum einen von der Komplexität (d. h. der Anzahl der möglichen Variablen der zu selektierenden Struktur), das Abnahmeverhältnis im Selektionsschritt, das im Rahmen der vorliegenden Erfindung zwischen 0,01 und 50 % liegt, sowie dem gegenläufigen Effekt der Mutation im Amplifikationsschritt bestimmt. Zusätzlich zu dem Selektionsschritt in (1) kann auch durch den Ablöseschritt (2)(e) eine zusätzliche Selektion erfolgen.

Das Verfahren (II) kann dabei weiterhin entweder nach jedem Evolutionszyklus oder nach dem letzten Evolutionszyklus weiterhin noch die zusätzlichen Schritte der Vereinzelung der erhaltenen Matrizenmoleküle und nachfolgende Analyse umfassen.

Die vorliegende Erfindung wird anhand der folgenden Figuren näher erläutert.

**Figurenbeschreibung**

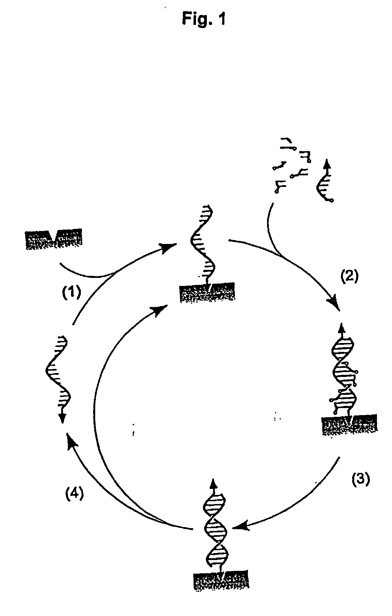

Figur 1: Allgemeines Schema des erfindungsgemäßen Verfahrens:

(1) Immobilisierungsschritt: Eine Matrize wird durch eine irreversible Reaktion mit der Oberfläche eines festen Trägers immobilisiert.
(2) Hybridisierungsschritt: Die Matrize bindet komplementäre Fragmente aus der Lösung.
(3) Ligationsschritt: Die Fragmente werden durch chemische Verknüpfung miteinander verknüpft.
(4) Ablösungsschritt: Die Kopie wird freigesetzt und an einem anderen Teil des festen Trägers erneut immobilisiert, wobei sie zu einer Matrize für den nächsten Cyelus von Schritten wird.

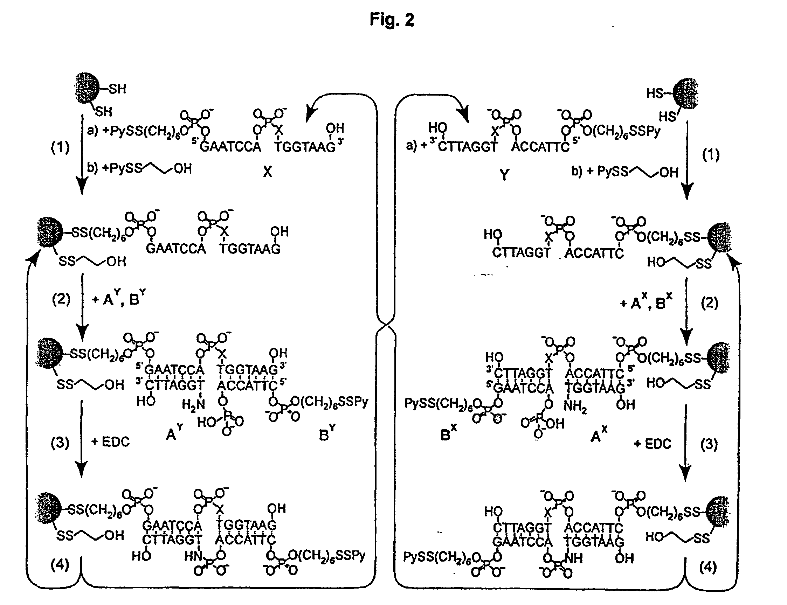

Figur 2: In dem Experiment eingesetzte Oligonucleotid-Analoga und Reaktionen:

Die einzelnen Schritte des Verfahrens (1) - (4) wurden für die komplementären Matrizen X und Y getrennt in verschiedenen Röhrchen durchgeführt. PySS bezeichnet eine 2-Pyridyldisulfid-Struktureinheit, die bei der Immobilisierung (1a) und Verkappung (1b) unter Bildung von 2-Thiopyridon gespalten wird. Die Gerüstmodifikation X an der zentralen Internucleotid-Verknüpfung von X und Y ist X = N-H, außer bei der ersten Immobilisierung, wo X = O ist. $A^x$, $B^x$, $A^y$ und $B^y$ bezeichnen die entsprechenden Matrizenfragmente. Der Hybridisierungsschritt (2) ergibt einen terriolekularen Komplex aus den immobilisierten Matrizen und den jeweiligen Fragmenten $A^x$, $B^x$, $A^y$ und $B^y$. In Gegenwart des wasserlöslichen Carbodiimids EDC führt die chemische Verknüpfung (3) zu einer 3'-5'-Phosphoramidat-Bindung zwischen einer 5'-Amino- und einer benachbarten 3'-Phosphatgruppe. Jeder resultierende doppelsträngige Komplex wird denaturiert (4), was den matrizentragenden Träger sowie eine auf frischem SH-Träger immobilisierte PySS-modifizierte Kopie ergibt.

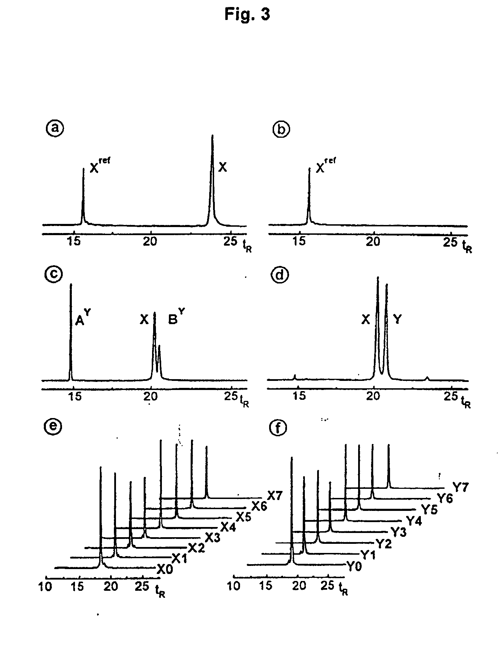

Figur 3: HPLC-Analyse von Produkten unter denaturierenden Bedingungen, die man in den aufeinanderfolgenden Schritten eines Amplifikations-Cyclus erhält. Alle HPLC-Proben ohne Reduktionspuffer (RB) wurden vor der Analyse auf eine Konzentration von 100 mM DTT gebracht (außer (a)), um die Reproduzierbarkeit der HPLC-Quantifizierung zu gewährleisten.

$t_R$ = Retentionszeit in Minuten

(a) = Reaktionsgemisch, das X und GAATCCATGGTAAG ($X^{ref}$) als internen Standard enthält, vor der Immobilisierung
(b) = Überstand nach der Immobilisierung
(c) = Reaktionsgemisch nach der Hybridisierung von immobilisiertem X mit $A^y$ und $B^y$ und Behandlung des Trägers mit RB. (d) Reaktionsgemisch nach der chemischen Verknüpfung und reduktive Abspaltung vom Träger mit Hilfe von RB
(e,f) = HPLC-Analyse der Gruppe von sechzehn Proben, die man durch reduktive Spaltung von immobilisierten Matrizen erhielt, die durch drei Amplifikations-Cyclen erzeugt wurden.

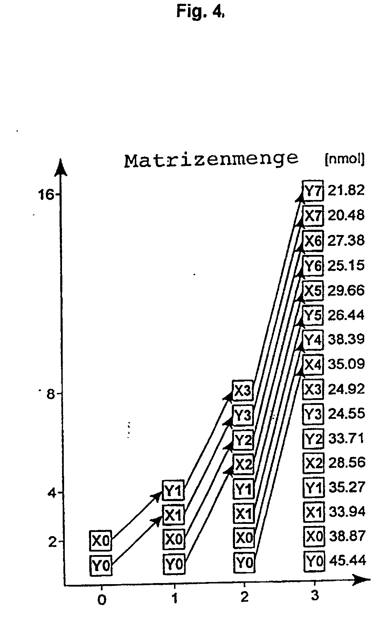

Die Beschriftungen rechts von den HPLC-Profilen ist nachfolgend in Figur 4 näher erläutert. Man beachte, daß kleine Verunreinigungen, die in den vorderen Profilen sichtbar sind, im Verlaufe der Amplifikation nach und nach verschwinden.

Figur 4: Weg der Matrizenübertragungen im Verlaufe von drei Amplifikations-Cyclen: Kästchen und Buchstaben symbolisieren Reaktionsröhrchen bzw. matrizentragende Träger. X-Achse: Zahl der Amplifikationszyklen; Y-Achse: Anzahl der Proben. Die Pfeile zeigen an, welche Kopie von welcher Matrize erzeugt wird. Die gezeigte Menge des Matrizenmaterials wurde nach der Spaltung der Disulfidbrücken durch Reduktion mit DTT durch HPLC-Analyse quantifiziert. Aus den obigen Daten wurden 14 individuelle Ausbeuten berechnet. Die mittleren Ausbeuten und Standardfehler sind: $p_x$ = 0.763 +/- 0.071 und $p_y$ = 0.855 +/- 0.079. Die Stoffmenge der jeweiligen Matrize beim Cyclus n in Nanomol wird mit ihrem theoretischen Wert (in eckigen Klammern) verglichen, der aus (3a,b) berechnet wird: $x_0$ = 38.87 (38.87), $y_0$ = 45.44 (45.44), $x_1$ = 72.81 (73.54), $y_1$ = 80.71 (78.68), $x_2$ = 126.29 (133.58), $y_2$ = 138.97 (141.57), $x_3$ = 238.90 (241.61), $y_3$ = 249.77 (255.80).

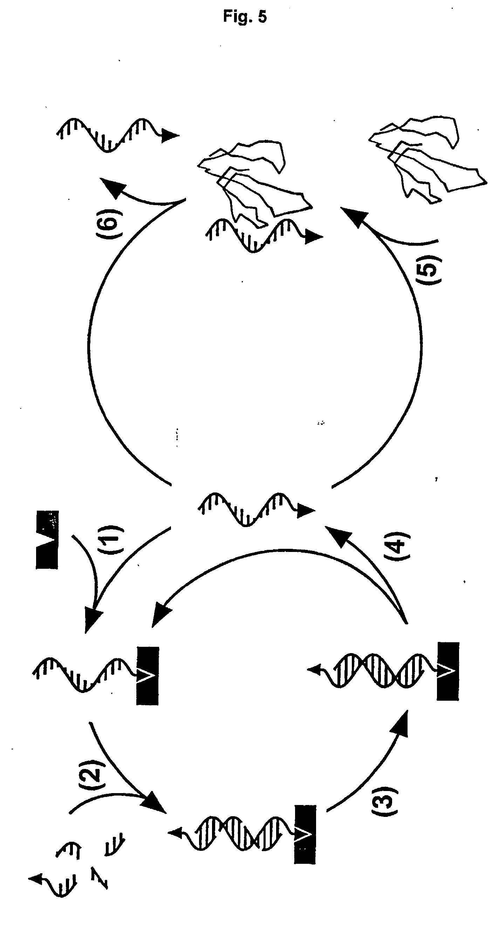

Figur 5: Allgemeines Schema des erfindungsgemäßen Verfahrens:

(1) Immobilisierungsschritt: Eine Matrize wird durch eine irreversible Reaktion mit der Oberfläche eines festen Trägers immobilisiert.
(2) Hybridisierungsschritt: Die Matrize bindet komplementäre Fragmente aus der Lösung.
(3) Ligationsschritt: Die Fragmente werden durch chemische Verknüpfung miteinander verknüpft.
(4) Ablösungsschritt: Die Kopie wird freigesetzt und an einem anderen Teil des festen Trägers erneut immobilisiert, wobei sie zu einer Matrize für den nächsten Cyclus von Schritten wird.

(5) Selektionsschritt: Anlagerung der Matrize an das Targetmolekül.

(6) Verwerfen der nichtbindenden Matrizen.

Im einzelnen beschreibt Figur 1 zwei besondere Ausführungsformen des Verfahrens (I) mit folgenden Schritten: (1) ein Templat-Immobilisierschritt, (2) ein Hybridisierungsoder Cappingschritt, (3) ein Ligations- oder Kopierschritt und (4) ein Ablösungs- oder Denaturierschritt. Es werden zwei Verfahrensvarianten (I und II) umfaßt, von denen (I) einen nicht-enzymatischen Kopierschritt und (II) einen enzymatischen Kopierschritt impliziert.

(I): In der ersten Verfahrensvariante werden als Templat Oligodesoxy- oder Oligoribonucleotidderivate verwendet, die 10-100 Nucleotideinheiten besitzen und entweder ein natürliches Rückgrat aus 3'-5'-Phosphoramidatbindungen, 3'-5'-Pyrophsophatbindungen oder anderen Bindungen einschließlich 2'-5'-Internucleotidbindungen enthalten. Natürliche und synthetische Bindungen können im Rückgrat gemischt vorliegen.

(ad 1) Die Immobilisierung des Templats erfolgt 5'-terminal über eine Disulfidbrücke oder über die Biotin-Avidin-Wechselwirkung. Im ersten Fall enthält das zu immobilisierende Oligonucleotid-Templat am 5'-Terminus eine 2-Pyridyldisulfidmodifikation und das Trägermaterial freie Thiolgruppen. Die Immobilisierung findet hier durch Disulfidaustausch unter Abspaltung von 2-Thiopyridon statt. Im zweiten Fall wird ein 5'-biotinyliertes Oligonucleotid-Templat eingesetzt, wobei der Biotin-Modifier auch eine Disulfidbrücke enthalten kann. Die Immobilisierung findet durch Bindung von Biotin an Avidin statt, das seinerseits an festen Trägern gebunden vorliegt. Als Trägermaterialien können Tentagele, Agarose oder andere geeignete Polymere einschließlich Glas oder Papier verwendet werden. (ad 2) Nach Immobilisierung werden die nicht-belegten Oberflächenstellen durch Behandlung mit einem geeigneten Reagenz desaktiviert. Thiolgruppen werden vorzugsweise durch Behandlung mit 2-Hydroxyethyl-(2-pyridyl)-disulfid, aber auch 2,2'-Dipyridyldisulfid, einem milden Alkylans, oder durch Oxidation desaktiviert. Freies Avidin wird mit Biotin desaktiviert.

(ad 3) Der Kopierschritt erfolgt durch chemische Ligation: 2-50-mere komplementäre Oligodesoxynucleotide, Oligoribonucleotide oder Abkömmlinge, die entweder eine 5'-Phosphat- und eine 3'-Hydroxygruppe oder eine 5'-Hydroxy- und eine 3'-Phosphatgruppe oder eine 5'-Phosphat- und eine 3'-Aminogruppe oder eine 5'-Amino- und eine 3'-Phosphatgruppe oder eine 5'- und eine 3'Phosphatgruppe oder weitere chemisch verknüpfbare Gruppen enthalten, werden an das immobilisierte Templat angelagert und in Gegenwart eines wasserlöslichen Carbodiimides, vorzugsweise EDC, oder eines anderen Kondensationsmittels wie Bromcyan oder N-Cyanoimidazol verknüpft. Das zum 3'-Ende der immobilisierten Matrize komplementäre Oligodesxoynucleotid besitzt die Funktion eines 5'-Primers. Es enthält am 5'-Ende eine der unter (1) genannten Modifikationen und am 3'-Ende eine der unter (3) genannten Gruppen.

(ad 4) Die Denaturierung unter Ablösung der Kopie erfolgt entweder durch Einstellen einer geeigneten hohen Temperatur oder durch Elution mit einer Hamstofflösung oder einem anderen Denaturierungsmittel.

Die abgelösten Kopien werden an frischem Träger- wie unter (1) beschrieben-- immobilisiert und dienen im nächsten Schritt als Matrize. Das neu belegte Trägermaterial wird mit demjenigen aus früheren Zyklen vereinigt. Immobilisierte (+)- und (-)-Stränge können getrennt oder gemeinsam vereinigt werden. Bei getrennter Vereinigung werden die Schritte (3) abwechselnd mit (+)- und (-)-Primern durchgeführt,; bei gemeinsamer Vereinigung wird jeder Schritt (3) mit beiden Primern durchgeführt.

(II): In der zweiten Verfahrensvariante entsprechen die Schritte (1), (3), (4) denen unter (I) genannten. Schritt (2) impliziert die enzymatische Verlängerung eines Primers oder die enzymatische Ligation von zwei oder mehreren Oligonucleotidfragmenten. Die zu amplifizierende Zielsequenz wird zunächst durch Verwendung eines 5'-modifizierten Primers in eine 5'-terminal immobilisierbare Sequenz umgeschrieben. Nach Immobilisierung des Templats erfolgt Hybridisierung mit dem 5'-modifizierten Primer und dessen Verlängerung durch dNPT in Gegenwart einer Polymerase. Alternativ kann hier eine Ligase-katalysierte Verknüpung von 5'-phosphorylierten Oligonukleotiden stattfinden.

Figur 4 faßt den Weg jedes einzelnen Trägers und die Menge des nach jedem Kopiercyclus erhaltenen Materials für das beschriebene Beispiel zusammen. Im allgemeinen braucht die Ausbeute p eines Replikationscyclus nicht notwendigerweise p = 1 zu erreichen, um einen exponentiellen Amplifikationsmodus zu ermöglichen. Im Falle palindromischer Matrizen, bei denen X = Y, $A^x = A^y$, $B^x = B^y$, ist die Menge des Materials $X_n$, die man nach n Replikationscyclen aus einer Anganfsmenge $x_0$ erhält, gegeben durch:

$$x_n = x_0 \cdot (1 + p)^n \tag{1},$$

was für p > 0 ein exponentielles Wachstum ermöglicht. Bei nichtpalindromischen Matrizen, die unseren Experimenten zugrundeliegen, besteht jede Replikationsrunde aus zwei Kopiercyclen mit den individuellen Ausbeuten $p_x$ und $p_y$ für die Synthese von X bzw. Y. Hier folgt die Menge der immobilisierten Matrizen X und Y der Iterationsfolge:

$$X_{n+1} = X_n + p_x \cdot y_n \text{ und } y_{n+1} = y_n + p_y \cdot x_n \tag{2a,b},$$

was für ein exponentielles Wachstum sowohl $p_x > 0$ als auch $p_y > 0$ erfordert. Die Gleichungen (2a,b) ergeben:

$$x_n = \frac{1}{2}\cdot\left(x_0 + y_0\cdot\sqrt{\frac{p_x}{p_y}}\right)\cdot\left(1 + \sqrt{p_x\cdot p_y}\right)^n + \frac{1}{2}\cdot\left(x_0 - y_0\cdot\sqrt{\frac{p_x}{p_y}}\right)\cdot\left(1 - \sqrt{p_x\cdot p_y}\right)^n$$

$$(3a,b),$$

$$y_n = \frac{1}{2}\cdot\left(y_0 + x_0\cdot\sqrt{\frac{p_y}{p_x}}\right)\cdot\left(1 + \sqrt{p_y\cdot p_x}\right)^n + \frac{1}{2}\cdot\left(y_0 - x_0\cdot\sqrt{\frac{p_y}{p_x}}\right)\cdot\left(1 - \sqrt{p_y\cdot p_x}\right)^n$$

wobei $x_0$ und $y_0$ die Anfangsmenge von X bzw. Y bezeichnen. Ein Vergleich der experimentellen und theoretischen Werte für $x_n$ und $y_n$, wie er in der Legende von Figur 4 angegeben ist, bestätigt, daß die in Figur 4 gezeigten Daten mit einem exponentiellen Modus der Amplifikation für Matrizenmaterialien im Einklang stehen.

Das Verfahren (I) kombiniert die Vorteile der Festphasenchemie mit der chemischen Replikation und kann für die nichtenzymatische und enzymatische Amplifikation von RNA, Peptiden und anderen Matrizen sowie für die Darwinsche Evolution in künstlichen chemischen Ökosystemen weiterentwickelt werden. Ähnliche Vorgänge haben vielleicht auch bei der Entstehung des Lebens auf der Erde eine Rolle gespielt, da sich die frühesten Replikationssysteme durch Ausbreiten auf mineralischen Oberflächen vermehrt haben könnten.

Weiterhin bildet das erfindungsgemäße Verfahren ein erstes Beispiel für exponentielle Amplifikation und Festphasenchemie bei der chemischen Replikation. Die Festphasenchemie ist eine Voraussetzung für eine praktikable Automatisierung des Verfahrens, da im wesentlichen nur Pipettier- und Filtrationsschritte beteiligt sind. Die exponentielle Amplifikation eröffnet die Möglichkeit einer gezielten molekularen Evolution. Im letzteren Zusammenhang hat das Verfahren das Potential einer steuerbaren Mutationshäufigkeit, da die Freisetzung der Kopien in Schritt (4) von der thermodynamischen Stabilität der immobilisierten Duplexe abhängen sollte und Mutanten damit schneller als perfekte Kopien eluiert werden sollten. Protokolle, die auf Temperaturcyclen (oder anderen Umgebungsparametern) sowie auf Lösungschemie beruhen, lassen sich weniger gut auf chemische Systeme anwenden, die auf kurzen Matrizen beruhen. Schnelle negative Temperatursprünge sowie eine schnelle Kondensationschemie wären notwendig, um die Reäquilibrierung der beteiligten Oligonucleotidkomplexe zu verhindern. Diese Bedingungen würden dann notwendigerweise die Genauigkeit des Matrizenkopierens beschränken, da die thermodynamische Kontrolle der molekularen Erkennung die Schwelle für die Kopiergenauigkeit in Abwesenheit eines energiedissipierenden Korrekturlesemechanismus festlegt.

Aus dem Vorstehenden ergibt sich unmittelbar, daß das erfindungsgemäße Verfahren von erheblichen Wert für den Entwurf von evolutionären chemischen Systemen ist. Andere Matrizen, Oberflächen, Matrizen-Oberflächen-Verknüpfungen können eingesetzt werden, enzymatische Varianten können erforscht werden, und verschiedene Schritte können miteinander kombiniert werden, so daß man zu einem höheren Niveau der Prozeßintegration und -autonomie gelangt. Es sind Protokolle denkbar, bei denen verwandte Matrizen nahe beieinander immobilisiert werden, so daß das Konzept der Quasispezies [M. Eigen, Naturwissenschaften 58, 465-523 (1971)] eine räumliche Dimension erhält. Weiterhin können Protokolle, bei denen zwei oder mehr verschiedene Klassen von Matrizenmolekülen, wie Oligonucleotide und Peptide, beteiligt sind, zur Selektion und Entdeckung von komplexeren kooperativen Systemen beitragen. Kurzfristig läßt sich das Verfahren bereits auf die Chemie der Peptid- und pRNA-Replikation anwenden, die von den Gruppen von D.H. Lee et al, Nature 382, 525-528 (1996) und B. Martin et al, Helv. Chim. Acta 80, 1901-1951 (1997) beschrieben wurden, sowie für die Synthese von sogenannten Spiegelmer-RNA (Jens Fürste et al., Nature Biotechnology 14, 1112, 1116; 15, 229).

Die Erfindung wird weiterhin anhand des nachfolgenden Beispiels näher erläutert.

## Beispiel

Materialien: Die Synthese der zwei komplementären 14-mere Matrizen, X und Y sowie der vier Matrizenfragmente, $A^x$, $B^x$, $A^y$ und $B^y$, erfolgte unter Verwendung von Standard-Phosphoramiditchemie, beispielsweise L. Beaucage et R. P. Iyer, Tetrahedron 48, 2223-2311 oder L. Beaucage et al., Tetrahedron Lett. 22, 1859 - 1862 (1981). Der thiolmodifizierter Träger wurde aus kommerziell erhältlicher 2-Pyridyldisulfid-aktivierter Agarose® (Sepharose® 6B, Pharmacia) durch Reduktion mit Dithiothreit (DTT) erhalten.

Reduktionspuffer (RB): 100 mM DTT, 40 mM Tris-HCl, 0,5 M NaCl, 1 mM EDTA, pH 7,9; Immobilisierungspuffer (IB):

370 mM Natriumacetat/Essigsäure, 0,5 M NaCl, 1 mM EDTA, pH 4,4, entgast mit Argon, um Sauerstoff auszutreiben. Verkappungspuffer (CB): 60 mM S-(2-Thiopyridyl)-2-mercaptoethanol, 40 mM MES, 0,5 M NaCl, 1 mM EDTA, pH 6,5. Hybridisierungspuffer (HB): 100 mM MES, 40 mM MgCl$_2$, 1 M NaCl, 1 mM 2,2'-Dipyridyldisulfid,pH 6,1. Verknüpfungspuffer (LB): HB mit 0,2 M EDC, frisch vor der chemischen Verknüpfung.

Allgemeine Verfahren: Wenn nichts anderes angegeben ist, wurden alle trägergestützten Reaktionen bei 25 °C in Mikro-Spin-Zentrifugenfiltern (0,5 ml, Celluloseacetat mit 0,45-µm-Poren, Roth) durchgeführt, die in Eppendorf-Röhrchen (1,5 ml) gestapelt waren. Jeder Filter einer Serie von 16 Filtern wurden mit 50 mg feuchter Sepharose® 6B (gemäß den Richtlinien des Herstellers gewaschen) beladen und dann zentrifugiert (3 min bei 4000 x g), um den Überstand zu entfernen. Vor einem Immobilisierungsschritt wurden die Kügelchen in 400 µl RB 1 h lang verquirlt, um die Thiolform von Sepharose 6B zu erzeugen. Dann wurde RB durch IB ersetzt, wobei die Zugabe von Puffer, Ultraschallbehandlung (10 s) und Zentrifugation sechsmal wiederholt wurden.

Immobilisierung: Eine Suspension von SH-Sepharose® in 400 µl IB, die 20-40 nmol der jeweiligen Matrize enthielt, wurde durch 1 h Verquirlen mit 1000 Ulmin gerührt.

Verkappen: 100 µl CB wurde zu der obigen Suspension gegeben. Nach 1 h Rühren wurden die Kügelchen durch Zentrifugation abgetrennt, in 400 µl CB erneut suspendiert und 1 h bei 25 °C verquirlt. Dann wurde CB durch eine Lösung von 200 mM S-(2-Thiopyridyl)-2-mercaptoethanol in Ethanol ersetzt. Nach 2 h Verquirlen bei 70 °C wurden die Kügelchen mit Ethanol (5 x 200 µl) und 1 M NaCl (5 x 200 µl) gewaschen.

Hybridisierung: Die Kügelchen wurden in 250 µl HB resuspendiert, der die komplementären Heptamere in einer Konzentration von 400 µM enthielt. Die Temperatur wurde auf 85 °C erhöht, innerhalb von 1 h auf 4 °C gesenkt und 2 h lang auf diesem Wert belassen. Überschüssige Heptamere wurden entfernt, indem man die Kügelchen mit drei 200-µl-Portionen 1 M NaCl von 4 °C wusch.

Chemische Verknüpfung: Die Kügelchen wurden in 375 µl LB resuspendiert, 40 h bei 4 °C verquirlt und dreimal mit 200 µl 1 M NaCl von 4 °C gewaschen.

Denaturierung: Ein Mikro-Spin-Filter mit den entsprechenden Kügelchen wurde in ein Eppendorf-Röhrchen eingesetzt, das 150 µl 1 M Acetatpuffer (pH 4,4) enthielt. Die Kügelchen wurden resuspendiert und in 50 µl 0,1 M NaOH leicht gerührt, und der Überstand wurde durch Zentrifugation (7000 x g, 30 s) in ein Eppendorf-Röhrchen übertragen. Nach dreimaligem Wiederholen dieses Verfahrensschrittes war die resultierende Lösung (400 µl) bereit für den nächsten Cyclus. Die Kügelchen wurden durch Waschen mit vier Portionen HB recycelt.

HPLC: Alle HPLC-Proben ohne Reduktionspuffer (RB) wurden vor der Analyse auf eine Konzentration von 100 mM DTT gebracht, um die Reproduzierbarkeit zu gewährleisten. Alle Trennungen wurden auf einer RP-C-18-Säule (250/4, Nucleosil® 120-5 AB, Macherey & Nagel) durchgeführt. Eluate: 0,1 M Triethylammoniumacetat (pH = 7)/MeCN 1% (A) und MeCN (B). Analytische Messungen wurden bei 50 °C vorgenommen, indem man einen Gradienten von 2% bis 6% A in 2 min, 6% bis 25% A in 30 min und eine Fließgeschwindigkeit von 1 ml/min verwendete. Das Eluat wurde gleichzeitig bei 254 nm und 273 nm überwacht. Ausrüstung (Kontron): zwei Pumpen 422, Autosampler 465, Flächenempfänger 440.Kroma 2000 wurde als Datenerfassungssystem verwendet.

Experiment: Zur Ausführung des in Figur 2 skizzierten Verfahrens wurde zunächst die 14-meren Matrizen X und Y unter Verwendung von Disulfid-Austauschreaktionen [J.R. Lorsch, Nature 371, 31-36 (1994)] jeweils auf eine Charge des SH-Trägers immobilisiert, wobei man die matrizentragenden Träger X0 und Y0 erhielt. Die Effizienz des Immobilisierungsschrittes wurde durch HPLC-Analyse des Überstandes bestimmt (Figur 3a,b). Die restlichen Thiolgruppen der Träger wurden durch Reaktion mit S-(2-Thiopyridyl)-2-mercaptoethanol verkappt. Dann wurden die Fragmente A$^x$, B$^x$ und A$^y$, B$^y$ mit den immobilisierten Matrizen Y0 bzw. X0 hybridisiert.

Zur Bestimmung der Hybridisierungseffizienz wurde ein Teil des Trägers mit DTT reduziert und durch HPLC analysiert (Figur 3c). Die chemische Verknüpfung [N.G. Dolinnaya et al., Nucleic Acids Res. 19, 3073-3080 (1991); K. D. James et al. Chem. Biol. 4, 595-605 (1997)] wurde erreicht, indem man den Hybridisierungspuffer gegen einen Verknüpfungspuffer austauschte, der N-Ethyl-N'-(dimethylaminopropyl)-carbodiimid-Hydrochlorid (EDC) als Kondensationsmittel enthielt.

Zur Bestimmung der Effizienz der Ligasierung wurde die Produktbildung durch die HPLC-Analyse des Produktgemischs überwacht, das man nach der Spaltung der Disulfidbindungen eines Aliquots der matrizentragenden Träger mit DTT als Reduktionsmittel erhielt (Figur 3d). Dann wurden die Kopien durch Spülen der Träger mit 0,1 M NaOH freigesetzt, durch HPLC analysiert und auf zwei neuen Chargen des SH-Trägers erneut immobilisiert, was die matrizentragenden Träger X1 (Kopie des matrizentragenden Trägers Y0) und Y1 (Kopie des matrizentragenden Trägers X0) ergab.

Für die nächste Generation wurde der gesamte Kreislauf von Schritten mit jeder der vier Chargen X0, Y0, X1 und Y1 wiederholt, was Y2, X2, Y3 bzw. X3 ergab. Eine weitere Runde ergab die matrizentragenden Träger X4..X7, Y4..Y7 (Figur 3e,f).

**Patentansprüche**

1. Verfahren zur exponentiellen Amplifikation molekularer Matrizen, umfassend

   (a) Binden von molekularen Matrizen an die Oberfläche einer Festphase mittels eines reversiblen Linkers an der Matrize;
   (b) Zugabe von Matrizenfragmenten, wobei eines der Fragmente eine gegebenenfalls geschützte Linkereinheit aufweist;
   (c) Synthese von Kopien der Matrize;
   (d) Entfernen von überschüssigen Matrizenfragmenten und Reaktionshilfstoffen;
   (e) Ablösen der Kopien von den Matrizen;
   (f) Besiedlung freier Bindungsstellen an der Festphase durch synthetisierte Matrizenkopien; und
   (g) Beliebig häufige Wiederholung der Schritte (a) - (f)

2. Verfahren gemäß Anspruch 1, wobei die in Schritt (e) erzeugte Matrizenkopie identisch oder komplementär zu den Ausgangsmatrizen sind.

3. Verfahren gemäß Anspruch (1), wobei zwei oder mehrere orthogonale Immobilisierungsverfahren an zwei oder mehreren Oberflächen zum Einsatz kommen.

4. Verfahren gemäß einem oder mehreren der Ansprüche 1 bis 3, wobei nach Schritt (a) überschüssige Bindungsstellen an der Festphase blockiert werden.

**5.** Verfahren gemäß einem oder mehreren der Ansprüche 1 bis 4, wobei die freien Bindungsstellen an der Festphase in Schritt (f) durch Freisetzen neuer Bindungsstellen an der Festphase, Zugabe neuen Festphasenmaterials oder Transfer der in (e) gebildeten Matrizenkopie auf neues Festphasenmaterial erzeugt werden.

**6.** Verfahren gemäß Anspruch 5, wobei die in Schritt (e) erzeugten Matrizen komplementär zu den Ausgangsmatrizen sind und auf neues Matrizenmaterial transferiert werden.

**7.** Verfahren gemäß einem oder mehreren der Ansprüche 1 bis 3, wobei die Linkereinheit des Matrizenfragments in Schritt (b) geschützt ist und die in Schritt (e) gebildeten Matrizenkopien entschützt werden.

**8.** Verfahren gemäß einem oder mehreren der Ansprüche 1 bis 7, wobei die Matrizen eine Länge von 2 bis 2000, insbesondere von 4 bis 50 Monomereinheiten. aufweisen und als Matrizenfragmente Monomereinheiten oder oligomere Monomereinheiten verwendet werden.

**9.** Verfahren gemäß Anspruch 1 oder 8, wobei die Menge in Schritt (a) eingesetzter Matrizen von $10^{-15}$ bis $10^{-1}$ mol/l beträgt und die Konzentration der Matrizenfragmente in Schritt (b) von $10^{-12}$ bis 1 mol/l beträgt.

**10.** Verfahren gemäß einem oder mehreren der Ansprüche 1 - 9, wobei die molekularen Matrizen ausgewählt sind aus Nukleotiden und Nukleotidabkömmlingen, Peptiden und Peptidabkömmlingen.

**11.** Verfahren gemäß einem oder mehreren der Ansprüche 1 - 10, wobei die in Schritt (c) gebildeten Bindungen ausgewählt sind aus Phosphorsäurediester-, Phosphorsäure-amidester-, Ester-, Amid-, Disulfid-, Acetal- und C-C-Bindungen.

**12.** Verfahren gemäß Anspruch 1 oder 11, wobei die Bindungsknüpfung in Schritt (c) chemisch oder durch enzymatische Verknüpfung erfolgt.

**13.** Verfahren gemäß einem oder mehreren der Ansprüche 1 - 12, wobei der Linker mittels kovalenter oder nichtkovalenter Bindungen an die Festphase bindet.

**14.** Verfahren gemäß einem oder mehreren der Ansprüche 1 - 13, wobei das Festphasenmaterial ausgewählt ist aus organischem oder anorganischem Material oder aus einem Hybrid dieser Materialien.

**15.** Verfahren zur chemischen Evolution durch exponentielle Amplifikation molekularer Matrizen, umfassend einen oder mehrere der Evolutionszyklen

(1) Selektion einer Subpopulation molekularer Matrizen aus einer kombinatorischen Ausgangsbibliothek; und
(2) Amplifikation und Mutation der selektiven Matrizen zur Bereitstellung einer Mutantenbibliothek, umfassend mehr als eine der in Ansprüchen 1 bis 14 definierten Amplifikationszyklen (a) bis (f).

**16.** Verfahren gemäß Anspruch 15, wobei die Selektion der Subpopulation molekularer Matrizenmoleküle in Schritt (1) durch eine reversible Bindung an eine oder mehrere Zielstrukturen erfolgt.

**17.** Verfahren gemäß Anspruch 15 oder 16, wobei die in Schritt (2) erhaltene Mutantenbibliothek die Ausgangsbibliothek für den nächsten Evolutionszyklus ist.

**18.** Verfahren gemäß einem oder mehreren der Ansprüche 15 - 17, wobei die Mutation durch die Reaktionsparameter in den Schritten (b) und (c) des Amplifikationszyklus gesteuert wird.

**19.** Verfahren gemäß einem oder mehreren der Ansprüche 15 - 18, wobei der erste Evolutionszyklus noch den folgenden Schritt (1') umfaßt: (1') Versehen der selektierten molekularen Matrizen mit einem reversiblen Linker.

**20.** Verfahren gemäß einem oder mehreren der Ansprüche 15 - 19, wobei in Ablöseschritt (e) durch selektives Ablösen eine zusätzliche Selektion erfolgt.

**Claims**

1. A process for the exponential amplification of molecular templates, comprising:

   (a) binding of molecular templates to the surface of a solid phase using a reversible linker on the template;

   (b) addition of template fragments wherein one of the fragments has an optionally protected linker unit;

   (c) synthesis of copies of the template;

   (d) removing of excess template fragments and reaction auxiliary agents;

   (e) detaching the copies from the templates;

   (f) colonization of free binding sites on the solid phase by synthesized template copies; and

   (g) repeating steps (a) to (f) for any number of times.

2. The process according to claim 1, wherein the template copies produced in step (e) are identical with or complementary to the starting templates.

3. The process according to claim 1, wherein two or more orthogonal immobilization methods are employed on two or more surfaces.

4. The process according to one or more of claims 1 to 3, wherein excess binding sites on the solid phase are blocked after step (a).

5. The process according to one or more of claims 1 to 4, wherein the free binding sites on the solid phase in step (f) are produced by exposing new binding sites on the solid phase, by the addition of new solid phase material, or by transferring the template copy formed in step (e) to a new solid phase material.

6. The process according to claim 5, wherein the templates produced in step (e) are complementary to the starting templates and are transferred to a new support material.

7. The process according to one or more of claims 1 to 3, wherein the linker unit of the template fragment in step (b) is protected and the template copies formed in step (e) are deprotected.

8. The process according to one or more of claims 1 to 7, wherein the templates have a length of from 2 to 2000, especially from 4 to 50, monomer units, and monomer units or oligomeric monomer units are used as said template fragments.

9. The process according to claim 1 or 8, wherein the amount of templates employed in step (a) is from $10^{-15}$ to $10^{-1}$ mol/l, and the concentration of the template fragments in step (b) is from $10^{-12}$ to 1 mol/l.

10. The process according to one or more of claims 1 to 9, wherein said molecular templates are selected from nucleotides and nucleotide derivatives, peptides and peptide derivatives.

11. The process according to one or more of claims 1 to 10, wherein the linkages formed in step (c) are selected from phosphoric acid diester, phosphoric acid amide ester, ester, amide, disulfide, acetal and C-C linkages.

12. The process according to claim 1 or 11, wherein the bond formation in step (c) is effected chemically or by enzymatic linking.

13. The process according to one or more of claims 1 to 12, wherein the linker binds to the solid phase through covalent bonds or non-covalent binding.

14. The process according to one or more of claims 1 to 13, wherein the solid-phase material is selected from organic or inorganic materials or from a hybrid of such materials.

**15.** A method of chemical evolution by the exponential amplification of molecular templates, comprising one or more of the following evolution cycles:

(1) selection of a subpopulation of molecular templates from a combinatorial starting library; and

(2) amplification and mutation of the selected templates to provide a mutant library comprising more than one of the amplification cycles (a) to (f) as defined in claims 1 to 14.

**16.** The method according to claim 15, wherein the selection of said subpopulation of molecular template molecules in step (1) is effected by reversible binding to one or more target structures.

**17.** The method according to claim 15 or 16, wherein the mutant library obtained in step (2) is the starting library for the next evolution cycle.

**18.** The method according to one or more of claims 15 to 17, wherein the mutation is controlled by the reaction parameters in steps (b) and (c) of the amplification cycle.

**19.** The method according to one or more of claims 15 to 18, wherein the first evolution cycle additionally comprises the following step (1'):

(1') providing the selected molecular templates with a reversible linker.

**20.** The method according to one or more of claims 15 to 19, wherein an additional selection is achieved in detaching step (e) by selective detaching.


**Revendications**

**1.** Procédé pour l'amplification exponentielle de matrices moléculaires, comprenant:

(a) la liaison de matrices moléculaires à la surface d'une phase fixe par l'intermédiaire d'un lien réversible à la matrice;
(b) l'addition de fragments de la matrice, l'un des fragments comportant une entité formant lien éventuellement protégée;
(c) la synthèse de copies de la matrice;
(d) l'écartement des adjuvants de la réaction et des fragments de matrice excédentaires,
(e) la séparation des copies des matrices;
(f) la multiplication des sites de liaison libres sur la phase fixe au moyen de copies de matrices synthétisées;

- le cas échéant une ou plusieurs répétitions des étapes (a) à (f).
- la répétition des étapes (a)-(f) autant de fois que souhaité.

**2.** Procédé selon la revendication 1, dans lequel, dans l'étape (e), les copies des matrices produites sont soit identiques aux matrices de départ soit complémentaires de celles-ci.

**3.** Procédé selon la revendication 1, dans lequel, interviennent deux ou plusieurs procédés d'immobilisation orthogonale à deux ou plusieurs autres surfaces.

**4.** Procédé selon une ou plusieurs des revendications 1 à 3, dans lequel, après l'étape (a) des sites de liaison excédentaires sur la phase fixe sont bloqués.

**5.** Procédé selon une ou plusieurs des revendications 1 à 4, dans lequel, les sites de liaison libres se forment sur la phase libre dans l'étape (f) par libération de nouveaux sites de liaison sur la phase fixe, addition de nouveaux matériaux de phase fixe ou transfert de la copie de matrice produite dans l'étape (e) sur un nouveau matériau de phase fixe.

**6.** Procédé selon la revendication 5, dans lequel, les matrices produites dans l'étape (e) sont complémentaires de la matrice de départ et sont transférées sur un nouveau matériau de matrice.

7.  Procédé selon une ou plusieurs des revendications 1 à 3, dans lequel, l'entité formant lien du fragment de matrice de l'étape (b) est protégée et les copies de matrices formées dans l'étape (e) sont déprotégés.

8.  Procédé selon une ou plusieurs des revendications 1 à 7, dans lequel, les matrices présentent une longueur correspondant à 2 à 2000, plus particulièrement 4 à 50 unités monomères et en ce que l'on utilise en tant que fragments de matrices des unités monomères ou des unités monomères oligomères.

9.  Procédé selon une ou plusieurs des revendications 1 à 8, dans lequel, 1a quantité de matrices introduite dans l'étape (a) est de $10^{-15}$ à $10^{-1}$ mol/l et la concentration des fragments de matrices dans l'étape (b) est de $10^{-12}$ à 1 mol/l.

10. Procédé selon une ou plusieurs des revendications 1 à 9, dans lequel, les matrices moléculaires sont choisies dans le groupe formé par les nucléotides, et les dérivés de nucléotides, les peptides et les dérivés de peptides.

11. Procédé selon une ou plusieurs des revendications 1 à 10, dans lequel les liaisons formées dans l'étape (c) sont choisies parmi les liaisons diester d'acide phosphorique, amido-ester d'acide phosphorique, esters, amides de disulfures, acétals ou C-C .

12. Procédé selon une ou plusieurs des revendications 1 à 11, dans lequel, les liens des liaisons dans l'étape (c) sont des liens enzymatiques ou chimiques.

13. Procédé selon une ou plusieurs des revendications 1 à 12, dans lequel, le lien se fixe à la phase fixe par l'intermédiaire d'une liaison covalente ou non covalente.

14. Procédé selon une ou plusieurs des revendications 1 à 13, dans lequel, la matériau de la phase fixe est choisi dans le groupe des matières organiques et inorganiques ou des hybrides de ces matières.

15. Procédé pour l'évolution chimique par amplification exponentielle de matrices moléculaires, comprenant un ou plusieurs des cycles d'évolution,

    (1) sélection d'une sous population de matrices moléculaires d'une bibliothèque de départ combinatoire;
    (2) amplification et mutation des matrices sélectives pour l'obtention d'une bibliothèque de mutants comprenant plus d'un cycle d'amplification (a) à (f) tels que définis dans les revendications 1 à 14.

16. Procédé selon la revendication 15, dans lequel, la sélection d'une sous population de matrices moléculaires dans l'étape (1) est réalisée par une liaison réversible sur une ou plusieurs structures cibles.

17. Procédé selon la revendication 15 ou 16, dans lequel, la banque de mutants obtenue à l'étape (2) est la bibliothèque de départ du cycle d'évolution suivant.

18. Procédé selon une ou plusieurs des revendications 15 à 17, dans lequel, la mutation est contrôlée par les paramètres réactionnels des étapes (b) et (c) du cycle d'amplification.

19. Procédé selon une ou plusieurs des revendications 15 à 18, dans lequel, le premier des cycles d'amplification comprend également l'étape (1') suivante:

    (1') disposition sur la matrice moléculaire sélective d'un lien réversible.

20. Procédé selon une ou plusieurs des revendications 15 à 19, dans lequel, l'étape de séparation (e) est réalisée par séparation sélective d'une sélection supplémentaire.

Fig. 1

Fig. 2

**Fig. 3**

**Fig. 4.**

Matrizenmenge [nmol]

| | | |
|---|---|---|
| Y7 | 21.82 |
| X7 | 20.48 |
| X6 | 27.38 |
| Y6 | 25.15 |
| X5 | 29.66 |
| Y5 | 26.44 |
| Y4 | 38.39 |
| X4 | 35.09 |
| X3 | 24.92 |
| Y3 | 24.55 |
| Y2 | 33.71 |
| X2 | 28.56 |
| Y1 | 35.27 |
| X1 | 33.94 |
| X0 | 38.87 |
| Y0 | 45.44 |

Fig. 5